# EUROPEAN PATENT APPLICATION

(11) **EP 4 773 078 A1**
(43) Date of publication of application: **08.07.2026**
(21) Application number: 25224454.6
(22) Date of filing: 17.12.2025
(51) Int. Cl.: G06T 7/00

(54) **METHOD FOR IMAGE-BASED CLINICAL DECISION SUPPORT FOR AXIAL SPONDYLOARTHRITIS USING ARTIFICIAL INTELLIGENCE**

(30) Priority: 07.01.2025 KR 20250002057
(71) Applicant: Samsung Life Public Welfare Foundation, Seoul 04348 (KR)
(72) Inventor: LEE, Seulkee, 06351 Seoul (KR); CHA, Hoon Suk, 06351 Seoul (KR); CHUNG, Myung Jin, 06351 Seoul (KR); YOO, Hakje, 06351 Seoul (KR); KIM, Yunseo, 06351 Seoul (KR)
(74) Representative: Plougmann Vingtoft a/s

(57) **Abstract**

The present invention relates to a method for generating diagnostic support information for axial spondyloarthritis. The method includes preprocessing an input MRI image through normalization and histogram matching. A first slice feature representing visual characteristics of the MRI image is extracted for each slice of the preprocessed MRI image by applying a first feature extraction model, and a second slice feature representing quantitative characteristics of bone marrow edema (BME) is extracted by applying a second feature extraction model to BME data associated with the MRI image. Slice-level integrated feature vectors are generated by combining the first and second slice features on a slice-by-slice basis and applying attention-based importance weights, and a patient-level final integrated feature vector is obtained by summing the weighted vectors. Diagnostic support information indicating whether axial spondyloarthritis is present is then generated by inputting the final integrated feature vector into a classification model.

## Description

### CROSS-REFERENCE TO RELATED APPLICATIONS

The present application is based on and claims priority to Korean Patent Application No. KR10-2025-0002057 filed on January 07, 2025, the entire contents of which are hereby incorporated by reference.

### Technical Field

This invention was carried out with the support of the Ministry of Health and Welfare under Project Unique Number 2460002042 and Sub-project Number 00439690, wherein the Korea Health Industry Development Institute served as the research management agency, the research program was entitled "Global Physician-Scientist Training," the research task was entitled "Identification of Biomarkers Predicting Biologic Therapeutic Response and Development of an Artificial Intelligence Model Through Single-Cell Analysis of Patients with Axial Spondyloarthritis," the performing institution was Samsung Medical Center, and the research period was from July 1, 2024 to December 31, 2024.

The present invention relates to a technique for generating diagnostic support information for axial spondyloarthritis, and more particularly, to a method and computing device for analyzing MRI images and Bone Marrow Edema (BME) data using deep learning to provide information indicative of axial spondyloarthritis for use by a clinician.

### Description of the Related Art

Axial spondyloarthritis (especially ankylosing spondylitis) is a chronic inflammatory disease where early diagnosis is important, and delays in proper treatment can lead to joint damage and dysfunction. The current diagnosis method is based on MRI to determine the presence or absence of Bone Marrow Edema (BME) according to an Assessment of Spondylarthritis International Society (ASAS) criterion. However, this conventional method has limitations in the reliability and consistency of diagnostic results, and in particular, reading MRI images depends heavily on the proficiency of medical staff, so subjective errors may occur.

In addition, the existing diagnosis method depending on the presence or absence of BME has a problem in that it is difficult to diagnose when BME does not appear due to chronic inflammation. As a result, the sensitivity and specificity of diagnosis are low, making it difficult to intervene early for patient treatment. In addition, since the process of reading MRI images takes a lot of time and effort, it is acting as a factor that increases the work burden of medical staff.

In order to solve this problem, the present invention proposes a technology that analyzes MRI and BME data in multi-modal using deep learning technology and provides objective and consistent diagnosis results. The present invention improves image quality in a pre-processing process, increases the diagnosis accuracy of axial spondyloarthritis through feature extraction and integrated analysis, and maximizes the efficiency of the diagnosis process. This is expected to reduce the burden on medical staff and provide faster and more accurate diagnosis to patients.

### Related Art Document

Japanese Patent No. 6994094

### Summary of Invention

### Problems to be Solved

Therefore, an object of the present invention is to provide a deep learning-based information generation method and computing device capable of analyzing MRI images and BME data to generate diagnostic support information related to axial spondyloarthritis, thereby assisting clinicians by improving the reliability and consistency of the interpretation process and reducing the time required for image review.

Another object of the present invention is to support clinical decision-making by reducing errors that may arise from BME dependence in existing approaches and by providing information indicative of axial spondyloarthritis even in cases where BME may not be clearly visible due to chronic inflammation.

### Means for Solving Problems

In order to achieve the above object, the present invention provides a method for assisting in the diagnosis of axial spondyloarthritis, the method comprising: preprocessing an input magnetic resonance imaging (MRI) image by performing normalization and histogram matching to standardize the MRI image; extracting, for each of a plurality of slices of the preprocessed MRI image, a first slice feature representing visual characteristics of the MRI image by applying a first feature extraction model; extracting a second slice feature representing quantitative characteristics of bone marrow edema (BME) by applying a second feature extraction model to BME data associated with the preprocessed MRI image; generating slice-level integrated feature vectors by combining, on a slice-by-slice basis, the first slice feature and the second slice feature, applying attention-based importance weights to the respective integrated feature vectors, and generating a patient-level final integrated feature vector by summing the weighted vectors; and generating diagnostic support information based on the patient-level final integrated feature vector for use by a clinician in making a diagnostic decision.

Preferably, the MRI image input to the first feature extraction model includes a fat-suppressed T1-weighted (FS T1-weighted) image and a short tau inversion recovery T2-weighted (STIR T2-weighted) image.

Preferably, the BME data input to the second feature extraction model comprises binary or quantitative data indicating the presence or a quantitative value of bone marrow edema (BME) labeled on the MRI image.

Preferably, the MRI image is preprocessed by performing contrast enhancement using contrast limited adaptive histogram equalization (CLAHE) after histogram matching.

Preferably, the first slice feature is generated by inputting each slice of the preprocessed MRI image into a convolutional neural network (CNN)-based first feature extraction model and extracting visual features of the MRI image through convolution operations and MBConv blocks of the model, the first slice feature comprising visual features associated with structural changes extracted from FS T1 slices and visual features associated with inflammatory changes extracted from STIR T2 slices.

Preferably, the second slice feature is generated by extracting quantitative characteristics of the BME data through multilayer perceptron (MLP) operations of the second feature extraction model.

Preferably, the generating diagnostic support information further comprises displaying or transmitting the diagnostic support information to a user through a display device, user interface, or external terminal.

Preferably, the generating diagnostic support information comprises causing the classification model to output a probability value indicating a likelihood of axial spondyloarthritis based on the patient-level final integrated feature vector, and providing diagnostic support information based on the probability value.

In addition, the present invention provides a non-transitory computer-readable medium storing instructions that, when executed by a processor, cause the processor to: perform normalization and histogram matching on an input magnetic resonance imaging (MRI) image to standardize the MRI image; extract, for each of a plurality of slices of the preprocessed MRI image, a first slice feature representing visual characteristics of the MRI image by applying a first feature extraction model; extract a second slice feature representing quantitative characteristics of bone marrow edema (BME) by applying a second feature extraction model to BME data associated with the preprocessed MRI image; generate slice-level integrated feature vectors by combining, on a slice-by-slice basis, the first slice feature and the second slice feature, applying attention-based importance weights to the integrated feature vectors, and generating a patient-level final integrated feature vector by summing the weighted vectors; and generate diagnostic support information based on the patient-level final integrated feature vector for use by a clinician in making a diagnostic decision.

Preferably, the diagnostic support information is displayed or transmitted to a user through a display, user interface, or external device.

### Effects of Invention

According to the present invention, there is an advantage in that diagnostic support information related to axial spondyloarthritis can be generated more reliably by analyzing MRI images and BME data using deep learning, thereby assisting clinicians in improving the consistency of image interpretation and reducing the time required for reviewing images. This contributes to enhancing efficiency in clinical workflows.

In addition, the present invention mitigates the problem of BME dependence in conventional approaches by providing information indicative of axial spondyloarthritis even when BME is not clearly observed due to chronic inflammation, thereby supporting clinicians in making more informed clinical decisions.

### Brief Description of Drawings

**FIG. 1** is a flowchart of a method for generating diagnostic support information for axial spondyloarthritis through artificial intelligence analysis according to an embodiment of the present invention.
**FIG. 2** illustrates a preprocessing procedure of a method for generating diagnostic support information for axial spondyloarthritis through artificial intelligence analysis according to an embodiment of the present invention.
**FIG. 3** is a flowchart illustrating a preprocessing step according to an embodiment of the present invention.
**FIG. 4** shows the structure of a feature extraction model used in a method for generating diagnostic support information for axial spondyloarthritis according to an embodiment of the present invention.
**FIG. 5** is a flowchart illustrating an integration and classification step of the method for generating diagnostic support information for axial spondyloarthritis according to an embodiment of the present invention.
**FIG. 6** is a block diagram of a computing device for generating diagnostic support information for axial spondyloarthritis through artificial intelligence analysis according to an embodiment of the present invention.
**FIG. 7** shows a classification output of the method for generating diagnostic support information for axial spondyloarthritis according to an embodiment of the present invention, illustrated using a confusion matrix.

### Details for Carrying Out the Invention

Hereinafter, the present invention will be described in detail with reference to the contents described in the accompanying drawings. However, the present disclosure is not limited by the exemplary embodiments. Like reference numerals presented in each drawing denote members that perform substantially the same function.

The objects and effects of the present disclosure may be naturally understood or become clearer by the following description, and the objects and effects of the present disclosure are not limited only by the following description. In addition, in describing the present disclosure, when it is determined that the detailed description of the known technology related to the present disclosure may unnecessarily obscure the gist of the present disclosure, the detailed description thereof will be omitted.

The terms used in the present invention are used only to describe specific embodiments, and are not intended to limit the present invention. Singular expressions include plural expressions unless the context clearly indicates otherwise. In the present application, it is to be understood that the terms such as "include" or "have" are intended to designate the presence of features, numbers, steps, operations, components, parts, or a combination thereof described in the description of the invention, and do not preclude the possibility of presence or addition of one or more other features, numbers, steps, operations, components, parts, or a combination thereof.

Terms such as first, second, etc. may be used to describe various components, but the components should not be limited by the terms. The terms are used only for the purpose of distinguishing one component from another. For example, without departing from the scope of the present disclosure, a first component may be referred to as a second component, and similarly, a second component may also be referred to as a first component.

Unless otherwise defined, all terms used herein, including technical or scientific terms, have the same meaning as those generally understood by those of ordinary skill in the art to which the present invention pertains. Terms such as those defined in commonly used dictionaries should be interpreted as having a meaning consistent with the meaning in the context of the related art, and unless clearly defined in the present invention, they are not interpreted as ideal or excessively formal meanings.

In interpreting the components, it is interpreted as including the error range even if there is no separate explicit description. The description of the temporal relationship includes, for example, a case in which the temporal precedence relationship is described as 'after', 'following', 'subsequently', 'before', etc., and a case in which the temporal precedence relationship is not continuous unless 'immediately' or 'directly' is used.

Hereinafter, the technical configuration of the present invention will be described in detail with reference to the accompanying drawings.

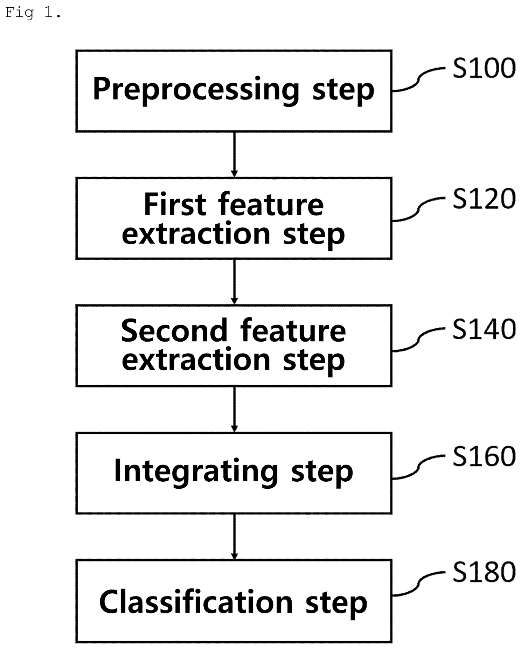

FIG. 1 is a flowchart of a method for generating diagnostic support information for axial spondyloarthritis through artificial intelligence analysis according to an embodiment of the present invention. Referring to FIG. 1, the method may include a preprocessing step S 100, a first feature extraction step S120, a second feature extraction step S140, an integrating step S160, and a classification step S180.

The method for generating diagnostic support information for axial spondyloarthritis may include a preprocessing step S100 of improving the contrast of the image by performing normalization and histogram matching to standardize the input MRI image. The method for generating diagnostic support information for axial spondyloarthritis may include a first feature extraction step S120 of extracting a visual feature of the MRI image from the preprocessed MRI image through a first feature extraction model. The method for generating diagnostic support information for axial spondyloarthritis may include a second feature extraction step S140 of extracting a quantitative feature of BME from a Bone Marrow Edema (BME) of a preprocessed MRI image through a second feature extraction model. The method for generating diagnostic support information for axial spondyloarthritis may include an integrating step S160 of generating an integrated feature vector using an integrated module that integrates the first feature and the second feature. The method for generating diagnostic support information for axial spondyloarthritis may include a classification step S180 of determining whether axial spondyloarthritis is present by inputting an integrated feature vector into a classification model.

The method for generating diagnostic support information for axial spondyloarthritis is designed with an end-to-end structure, and reliability of the generated diagnostic support information is improved by utilizing both image and BME modality data. The method for generating diagnostic support information for axial spondyloarthritis applies medical image-specific enhancement techniques such as horizontal inversion, rotation, color deformation (Affine, Perspective), and gamma change to prevent data overfitting and improve the generalization of the model.

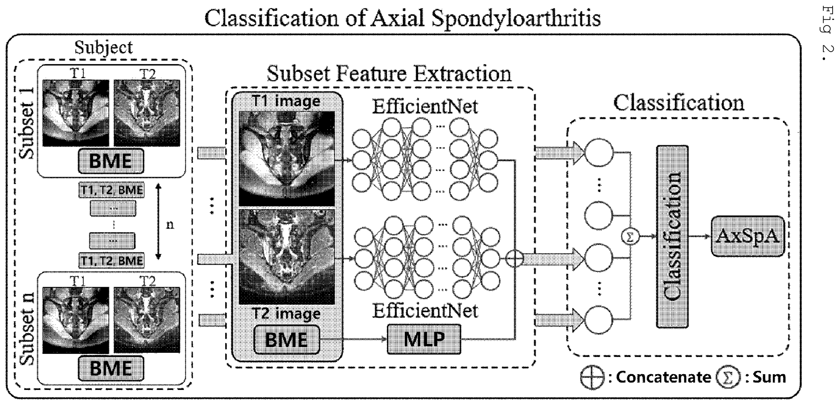

FIG. 2 is a flowchart of a preprocessing step according to an embodiment of the present invention. Referring to FIG. 2, in the method for generating diagnostic support information for axial spondyloarthritis, for generating information indicative of axial spondyloarthritis, patient-level information is analyzed using various data sources in a multi-modal method.

Specifically, the method for generating diagnostic support information for axial spondyloarthritis is grouped into n 'Subset' based on each MRI slice and processed. The Subset may include a FS T1 image, a STIR T2 image, and data of BME information, and may include a preprocessing step S100 of defining the entire data of the patient as 'Subject'. The method for generating diagnostic support information for axial spondyloarthritis may include a first feature extraction step S120 in which the T1 and T2 images in each Subset extract features through an CNN-based classification model (EfficientNet), and the BME information may include a second feature extraction step S140 in which meaningful features are derived by using a multi-layer percecptron (MLP).

Various features extracted from the method for generating diagnostic support information for axial spondyloarthritis may include an integrating step S160 of combining into one through a concatenation operation in each Subset. In the method for generating diagnostic support information for axial spondyloarthritis, the integrating step S160 effectively reflects the patient's multidimensional information to capture various patterns necessary for diagnosing axial spondyloarthritis. The method for generating diagnostic support information for axial spondyloarthritis may include an integrating step S160 of integrally learning the combined features of each Subset through a Attention technique. In the integrating step S160, the model intensively learns important information among several slices, and through this, a summary and emphasis of the overall information may be made. The method for generating diagnostic support information for axial spondyloarthritis may include a classification step S180 of generating classification-based information indicative of axial spondyloarthritis by assigning weights to important features in the Sum operation through the Attention technique.

The method for generating diagnostic support information for axial spondyloarthritis may improve the reliability of diagnostic support information by integrating various information obtained from several MRI slices and learning differentially according to the importance of each information.

The MRI image input in the method for generating diagnostic support information for axial spondyloarthritis includes a FS T1 (Fat-Suppressed T1-weighted) image in a coronal direction, and a STIR (Short Tau Inversion Recovery) T2 image. In the MRI image input in the method for generating diagnostic support information for axial spondyloarthritis, the presence or absence of BME (Bone Marrow Edema) is labeled. The input MRI image FS T1 MRI shows a structural change well, and the input MRI image STIR T2 MRI has a characteristic of effectively visualizing an inflammatory change.

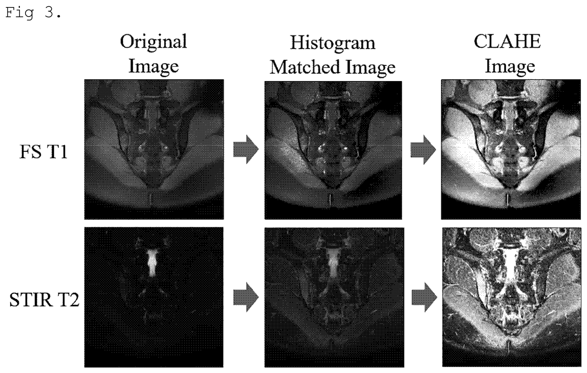

FIG. 3 illustrates a preprocessing process of the method for generating diagnostic support information for axial spondyloarthritis through artificial intelligence analysis according to an embodiment of the present invention. Referring to FIG. 3, in the preprocessing step S100, the first row is a ceiling joint image, the first row is an FS T1 image, and the second row is a STIR T2 image, and each image is preprocessed. The preprocessing step S100 may include performing a normalization technique to standardize data for each MRI slice, and the preprocessing step S100 may perform a Contrast Limited Adaptive Histogram Equalization (CLAHE) after a histogram match to improve image contrast.

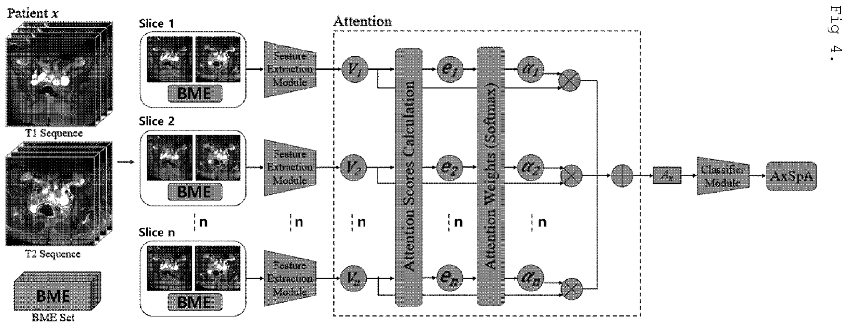

FIG. 4 shows the structure of a model of a feature extraction step of a method for generating diagnostic support information for axial spondyloarthritis through artificial intelligence analysis according to an embodiment of the present invention. Referring to FIG. 4, the feature extraction step includes a first feature extraction step S 120 and a second feature extraction step S140. The first feature extraction step S120 may extract a feature for each slice of the FS T1 and STIR T2 images using EfficientNet b4, and the second feature extraction step S140 may extract a quantitative feature of the BME data through Multi-Layer Perceptron (MLP).

In the first feature extraction step S120, visual features of the MRI image may be extracted from the preprocessed MRI image through the first feature extraction model. In the first feature extraction step S120, the preprocessed MRI image may be input to the first feature extraction model of the CNN series, and the visual feature of the preprocessed MRI image through the Convolution operation and MBConv block of the first feature extraction model may be extracted, but the visual feature related to the structural change may be extracted from the FS T1 image, and the visual feature related to the inflammatory change may be extracted from the STIR T2 image.

Specifically, in the first feature extraction step S120, the FS T1 image, the STIR T2 image, and the BME data are received as inputs, and respective features are extracted to generate respective feature vectors. In the first feature extraction step S120, the FS T1 image and the STIR T2 image are transmitted to the EfficientNet-based network to extract unique features of each image. In the first feature extraction step S120, the first feature extraction model, EfficientNet structure starts with an initial 3x3 Convolution layer, and several MBConv blocks are stacked thereafter. In the first feature extraction model, the MBConv block analyzes the input image from various angles using 3x3 and 5x5 kernels, and through this process, a V_{T1} vector is generated from the T1 image and a V_{T2} vector is generated from the T2 image. The first feature extraction model is designed to effectively learn the unique characteristics of each modality. The first feature extraction model is not limited to the EfficientNet-based network, and may extract features through various convolutional-based neural networks including a Convolutional Neural Network (CNN)-based network.

In the second feature extraction step S140, a quantitative feature of the BME may be extracted from the BME (Bone Marrow Edema) data associated with the preprocessed MRI image through the second feature extraction model. In the second feature extraction step S140, a quantitative feature of the BME data may be extracted through a Multi-Layer Perceptron (MLP) operation of the second feature extraction model.

Specifically, in the second feature extraction step S140, BME data composed of binary data may be used as an input, and in the second feature extraction step S140, the second feature extraction model may extract a feature of binary data. The second feature extraction model may be a MLP composed of three Layer. The second feature extraction model may include an input layer, a hidden layer, and an output layer, and through this process, an V_{BME} vector may be generated by extracting the characteristics of the BME. This process expresses the BME data in a high-dimensional manner and converts the data into a form that can be combined with other modalities. The second feature extraction model includes, but is not limited to, MLP, and may be implemented in various neural network structures that perform nonlinear data analysis and quantitative feature extraction.

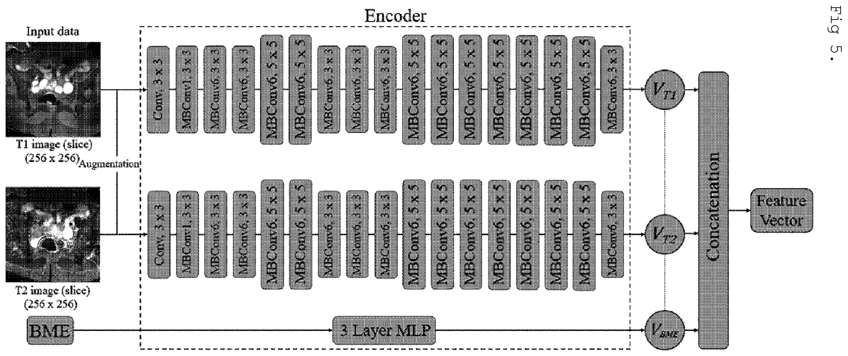

FIG. 5 is a flowchart illustrating an integration and classification step of the method for generating diagnostic support information for axial spondyloarthritis through artificial intelligence analysis according to an embodiment of the present invention. Referring to FIG. 5, an integrating step S160 is partially included. In the integrating step S160, the V_{T1} extracted from the T1 image, the V_{T2} extracted from the T2 image, and the V_{BME} extracted from the BME are integrated into one feature vector through a concatenation process. In the integrating step S160, the integrated vector fuses information of each modality to provide important multidimensional data for generating information relevant to axial spondyloarthritis analysis.

In the integrating step S160, each Subset extracts a feature of each modality through a feature extraction module. In the integrating step S160, the features extracted from the MRI data and the features extracted from the BME-related quantitative data are combined into one vector to integrate the information of each slice.

The feature extraction step includes an integrated module that individually learns the characteristics of each modality and finally integrates them to generate one integrated information vector, and enhances the quality of information used for axial spondyloarthritis-related analysis. The feature extraction step may be a feature extraction module.

In the integrating step S160, an integrated feature vector may be generated using an integrated module that integrates the first feature and the second feature. Based on this, in the classification step S180, a classification output related to axial spondyloarthritis is generated by inputting the integrated feature vector into the classification model, and a likelihood or indicator associated with axial spondyloarthritis is produced.

Specifically, in the integrating step S160, the integration module may be applied to integrate the features into one vector, and the integration module may assign weights to the first feature and the second feature according to the importance of each slice, and the weights may be reflected. In the integrating step S160, integrated information for each slice of the MRI data is analyzed using the Attention technique.

In the integrating step S160, the integrated vector is provided to an attention module to calculate the importance of each slice. The weight calculation module calculates a value indicating the importance of the vector, and then a final weight is derived through a normalization process. The weights reflect the relative importance of each slice, and the important slices are given higher weights. In the integrating step S160, the weighted feature vectors are summed for each slice to generate a final output vector for the corresponding MRI data instance.

Specifically, in the classification step S180, the classification model generates an output related to axial spondyloarthritis by analyzing the feature vector input through a deep learning-based classification algorithm. The classification model may produce a classification output, which may be expressed in the form of a probability value indicating a likelihood associated with axial spondyloarthritis.

In the classification step S180, the weighted feature vector is provided to a classifier module, which generates an output indicating a probability, score, or indicator associated with axial spondyloarthritis based on the integrated features.

This process generates processed information derived from multiple data modalities and enhances the quality of analysis by combining and evaluating multimodal features, thereby reducing limitations inherent in using a single data source.

In addition, the classification output or indicator generated in step S180 may be displayed through a display device or transmitted to an external terminal or user interface, thereby making the processed information accessible for clinical review or further analysis.

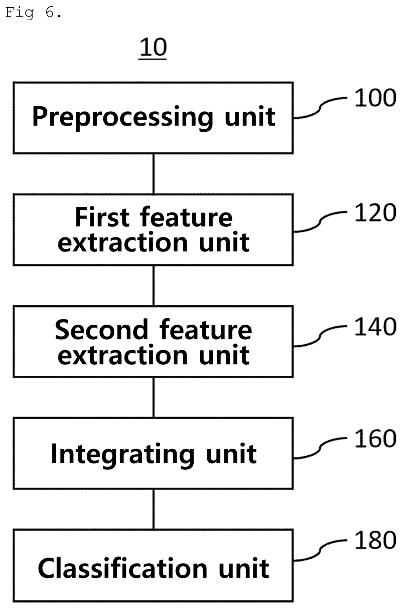

FIG. 6 is a block diagram of a computing device for generating diagnostic support information for axial spondyloarthritis through artificial intelligence analysis according to an embodiment of the present invention. Referring to FIG. 6, the computing device 10 for generating diagnostic support information for axial spondyloarthritis may include a preprocessing unit 100, a first feature extraction unit 120, a second feature extraction unit 140, an integration unit 160, and a classification unit 180.

As used herein, a component, module, or unit may include routines, procedures, programs, components, data structures, or the like that perform specific tasks or implement particular abstract data types. Those skilled in the art will also recognize that the methods described in the present disclosure may be implemented in various computer system configurations, including single-processor or multiprocessor computer devices, minicomputers, mainframe computers, personal computers, handheld computing devices, microprocessor-based or programmable consumer electronics, and the like. Each of these devices may be operated in association with one or more connected peripheral devices.

The embodiments described herein may also be implemented in distributed computing environments, in which tasks are performed by remote processing devices that are linked through a communication network. In such distributed computing environments, program modules may be located in both local and remote memory storage devices.

A computing device typically includes various computer-readable media. Any medium that can be accessed by a computer may be a computer-readable medium, and such media include both volatile and non-volatile media, transitory and non-transitory media, and removable and non-removable media. By way of example, computer-readable media may include both computer-readable storage media and computer-readable transmission media.

Computer-readable storage media include volatile and non-volatile media, transitory and non-transitory media, and removable and non-removable media implemented in any method or technology for storing information such as computer-readable instructions, data structures, program modules, or other data. Computer-readable storage media may include, but are not limited to, RAM, ROM, EEPROM, flash memory or other memory technology, CD-ROM, DVD or other optical disk storage, magnetic cassettes, magnetic tape, magnetic disk storage, or other magnetic storage devices, and any other medium that can be accessed by a computer and used to store desired information.

Computer-readable transmission media, on the other hand, include a modulated data signal such as a carrier wave or other transport mechanism that implements computer-readable instructions, data structures, program modules, or other data, and include any information transmission media. The term "modulated data signal" means a signal that has one or more of its characteristics set or changed in such a manner as to encode information within the signal.

Computer-readable transmission media may include wired media such as a wired network or direct-wired connection, and wireless media such as acoustic, RF, infrared, or other wireless media. Any combination of the aforementioned media may also be included within the scope of computer-readable transmission media.

The preprocessing unit 100 may improve the contrast of the image by performing normalization and histogram matching to standardize the input MRI image. The preprocessing unit means the preprocessing step S 100 described above.

The first feature extraction unit 120 may extract a visual feature of the MRI image from the preprocessed MRI image through the first feature extraction model. The first feature extraction unit means the above-described first feature extraction step S 120.

The second feature extraction unit 140 may extract a quantitative feature of the BME from the BME (Bon Marrow Edema) of the preprocessed MRI image through the second feature extraction model. The second feature extraction unit means the second feature extraction step S140 described above.

The integrating unit 160 may generate an integrated feature vector by using an integration module that integrates the first feature and the second feature. The integrating unit means the integrating step S160 described above.

The classification unit 180 may input the integrated feature vector to a classification model to generate an output indicating a likelihood or indicator associated with axial spondyloarthritis. The classification unit corresponds to the classification step S180 described above.

In addition, the classification unit 180 may display or transmit the generated classification information to an external device or user interface.

The computing device may further include an output module or interface configured to display the generated diagnostic support information on a display device or to transmit the information to an external system or user terminal.

### Example 1: Prediction Results of a Method for generating diagnostic support information for axial spondyloarthritis

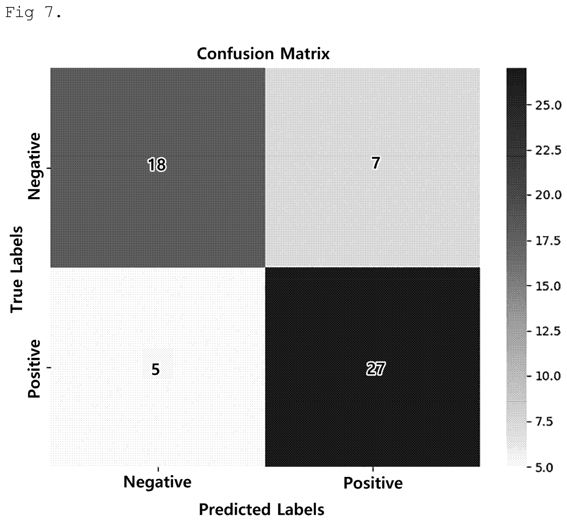

FIG. 7 shows a classification output of the method for generating diagnostic support information for axial spondyloarthritis through artificial intelligence analysis according to an embodiment of the present invention through a confusion matrix.

For performance evaluation, learning and testing were performed using data from a total of 291 people. 234 people were used for the training dataset, and 57 people were used for the test dataset, and it was confirmed that the accuracy of the test result was 78.95%, the sensitivity was 84.38%, the specificity was 72%, and the area under curve (AUC) was 86.00%. This result shows that the present invention is clinically applicable.

Although the present invention has been described in detail through the exemplary embodiments above, those skilled in the art to which the present invention belongs will understand that various modifications are possible to the above-described embodiments within the scope of the present invention. Therefore, the scope of the present invention should not be limited to the described embodiments, but should be defined by all changes or modifications derived from the concept of patent claims and the concept of equality as well as the scope of patent claims to be described later.

### Description of Reference Numerals

10: computing device
100: Preprocessing unit
120: First feature extraction unit
140: Second feature extraction unit
160: Integration unit
180: Classification unit

## Claims

1. A method performed by a computer device for generating information for assisting in the diagnosis of axial spondyloarthritis, the method comprising:
preprocessing an input magnetic resonance imaging (MRI) image by performing normalization and histogram matching to standardize the MRI image;
extracting, for each of a plurality of slices of the preprocessed MRI image, a first slice feature representing visual characteristics of the MRI image by applying a first feature extraction model;
extracting a second slice feature representing quantitative characteristics of bone marrow edema (BME) by applying a second feature extraction model to BME data associated with the preprocessed MRI image;
generating slice-level integrated feature vectors by combining, on a slice-by-slice basis, the first slice feature and the second slice feature, applying attention-based importance weights to the respective integrated feature vectors, and generating a patient-level final integrated feature vector by summing the weighted vectors; and
generating diagnostic support information based on the patient-level final integrated feature vector for use by a clinician in making a diagnostic decision.

2. The method of claim 1,
wherein the MRI image input to the first feature extraction model includes a fat-suppressed T1-weighted (FS T1-weighted) image and a short tau inversion recovery T2-weighted (STIR T2-weighted) image.

3. The method of claim 1,
wherein the BME data input to the second feature extraction model comprises binary or quantitative data indicating the presence or a quantitative value of bone marrow edema (BME) labeled on the MRI image.

4. The method of claim 1,
wherein the MRI image is preprocessed by performing contrast enhancement using contrast limited adaptive histogram equalization (CLAHE) after histogram matching.

5. The method of claim 1,
wherein the first slice feature is generated by inputting each slice of the preprocessed MRI image into a convolutional neural network (CNN)-based first feature extraction model and extracting visual features of the MRI image through convolution operations and MBConv blocks of the model, the first slice feature comprising visual features associated with structural changes extracted from FS T1 slices and visual features associated with inflammatory changes extracted from STIR T2 slices.

6. The method of claim 1,
wherein the second slice feature is generated by extracting quantitative characteristics of the BME data through multilayer perceptron (MLP) operations of the second feature extraction model.

7. The method of claim 1,
wherein the generating diagnostic support information further comprises displaying or transmitting the diagnostic support information to a user through a display device, user interface, or external terminal.

8. The method of claim 1,
wherein the generating diagnostic support information comprises causing the classification model to output a probability value indicating a likelihood of axial spondyloarthritis based on the patient-level final integrated feature vector, and providing diagnostic support information based on the probability value.

9. A non-transitory computer-readable medium storing instructions that, when executed by a processor, cause the processor to:
perform normalization and histogram matching on an input magnetic resonance imaging (MRI) image to standardize the MRI image;
extract, for each of a plurality of slices of the preprocessed MRI image, a first slice feature representing visual characteristics of the MRI image by applying a first feature extraction model;
extract a second slice feature representing quantitative characteristics of bone marrow edema (BME) by applying a second feature extraction model to BME data associated with the preprocessed MRI image;
generate slice-level integrated feature vectors by combining, on a slice-by-slice basis, the first slice feature and the second slice feature, applying attention-based importance weights to the integrated feature vectors, and generating a patient-level final integrated feature vector by summing the weighted vectors; and
generate diagnostic support information based on the patient-level final integrated feature vector for use by a clinician in making a diagnostic decision.

10. The non-transitory computer-readable medium of claim 9,
wherein the diagnostic support information is displayed or transmitted to a user through a display, user interface, or external device.
